(19) Europäisches Patentamt
European Patent Office
Office européen des brevets

(11) **EP 4 779 030 A1**

(12) **EUROPEAN PATENT APPLICATION**
published in accordance with Art. 153(4) EPC

(43) Date of publication:
**22.07.2026 Bulletin 2026/30**

(21) Application number: **24856552.5**

(22) Date of filing: **12.09.2024**

(51) International Patent Classification (IPC):
**C12Q 1/6809** (2018.01)    **G16H 10/40** (2018.01)

(52) Cooperative Patent Classification (CPC):
**C12Q 1/68; C12Q 1/6809; C12Q 1/6827;
G16H 10/40**

(86) International application number:
**PCT/JP2024/032774**

(87) International publication number:
**WO 2025/041864 (27.02.2025 Gazette 2025/09)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.08.2023 JP 2023134891**

(71) Applicant: **Seedna Inc.
Tokyo 121-0813 (JP)**

(72) Inventor: **TOMIKANE, Kinori
Tokyo 121-0813 (JP)**

(74) Representative: **dompatent
Partnerschaft von
Patentanwälten und Rechtsanwälten mbB
Deichmannhaus am Dom
Bahnhofsvorplatz 1
50667 Köln (DE)**

(54) **NONINVASIVE PRENATAL PATERNITY APPRAISAL METHOD**

(57)     A first object of the invention is to provide a novel non-invasive prenatal paternity testing method that enables the genotyping of a fetus using simple criteria.

The invention for solving the first problem is a non-invasive prenatal paternity testing method including performing a two-sample testing step by performing the genotyping of a fetus according to a determination method based on criterion a or performing a three-sample testing step by performing the genotyping of a fetus by a determination method based on criterion a or a determination method based on criterion b.

FIG. 1

# EP 4 779 030 A1

**Description**

Technical Field

The present invention relates to a technique for a non-invasive prenatal paternity test.

Background Art

A technique for testing paternity of a fetus before birth and an alleged father based on analysis results of circulating cell-free DNA (cfDNA) mixed in blood has been known. Analysis of circulating cell-free fetal DNA (cffDNA), which is a genetic material derived from a fetus and mixed in the blood circulation of a mother, enables performing a non-invasive prenatal paternity test (NIPPT) (for example, Patent Literature 1).

Citation List

Patent Literature

Patent Literature 1: JP 2014-502845 A

Summary of Invention

Technical Problem

In the non-invasive prenatal parentage test, the genotype of a fetus is identified based on the analysis result of a cfDNA sample. However, in cfDNA, DNA derived from a mother and DNA derived from a fetus are mixed. Because of this, in the signal measured from a single polymorphic locus, a signal from an allele derived from the mother and a signal from an allele derived from the fetus are mixed. For perform the genotyping of a fetus, it is necessary to estimate the signal of an allele derived from a fetus from the mixed signals. Thus, a first object of the invention is to provide a novel non-invasive prenatal paternity testing method that enables the genotyping of a fetus using simple criteria.

Solution to Problem

The invention for solving the first problem is as follows.

[1] A non-invasive prenatal paternity testing method, including
a two-sample testing step and/or a three-sample testing step defined below.

<Two-sample Testing Step>

A step of determining whether or not a biological parent-child relationship exists between an alleged father and a fetus based on the genotyping results of two persons: the alleged father and the fetus, at a plurality of polymorphic loci.
Note that the genotyping of the fetus is performed according to the determination method based on criterion a, defined below.

(Determination Method Based on Criterion a)

Provided that

the allele which is contained in data obtained by analysis of a circulating cell-free nucleic acid sample collected from a pregnant mother, derived from a specified polymorphic locus and detected with the strongest signal intensity, is denoted as allele A, and
the allele detected with the same signal intensity as that of allele A or the second strongest signal intensity is denoted as allele B,
determination is performed using the ratio of the signal intensity (Fa) of allele A and the ratio of the signal intensity (Fb) of allele B with respect to the combined intensity of signals derived from the specified polymorphic locus, as indexes, and according to the following criterion a.

<Criterion a>

· If 0% ≤ Fb {≤, <} L% is satisfied, the genotype at the specified polymorphic locus of a fetus is determined to be AA homozygote
· If M% {≤, <} Fb {≤, <} N% is satisfied, the genotype at the specified polymorphic locus of a fetus is determined to be AB heterozygote
· If Fa = Fb is satisfied, the genotyping of the specified polymorphic locus is skipped without being performed
· If Fb does not satisfy any of the above conditions, the genotyping of the specified polymorphic locus is skipped without being performed

(N is a numerical value of less than 50 and satisfies the relational expression of 0 {≤, <} L < M {≤, <} N. Note that the notation of {≤, <} indicates that either one of the inequality signs, ≤ and <, can be arbitrarily selected).

<Three-sample Testing Step>

A step of determining whether or not a biological parent-child relationship exists between an alleged father and a fetus based on the genotyping results of a biological mother, the alleged father, and the fetus at a plurality of polymorphic loci.

Note that the genotyping of the fetus is performed according to the determination method based on criterion a and/or the determination method based on criterion b, defined below.

(Determination Method Based on Criterion b)

Provided that

the allele which is contained in data obtained by analysis of a circulating cell-free nucleic acid sample, derived from a specified polymorphic locus determined as a homozygote in the mother, and detected with the strongest signal intensity, is denoted as allele A, and
the allele which is detected with the same signal intensity as allele A or the second strongest signal intensity is denoted as allele B,
determination is performed using the ratio of the signal intensity (Fa) of allele A and the ratio of the signal intensity (Fb) of allele B with respect to the combined intensity of signals derived from the specified polymorphic locus, as indexes, and according to the following criterion b.

<Criterion b>

· If 0% ≤ Fb {≤, <} L% is satisfied, the genotype at the specified polymorphic locus of a fetus is determined to be AA homozygote
· if M% {≤, <} Fb is satisfied, then the genotype at the specified polymorphic locus of the fetus is determined to be AB heterozygote
· If Fa = Fb is satisfied, the genotyping of the specified polymorphic locus is skipped without being performed
· If Fb does not satisfy any of the above conditions, the genotyping of the specified polymorphic locus is skipped without being performed

(Relational expression of (0 {≤, <} L < M is satisfied. Note that the notation of {≤, <} indicates that either one of the inequality signs, ≤ and <, can be arbitrarily selected).
[2] The non-invasive prenatal paternity determination method according to [1], wherein the testing whether or not a biological parent-child relationship exists includes
obtaining a combined paternity index (CPI) by multiplying paternity indexes (PI) obtained for individual polymorphic loci, thereby determining "positive", "negative", or "undeterminable" based on a criterion determined in advance according to the numerical value of the CPI,
PI is obtained based on the following Table 1, in the two-sample testing step, and
PI is obtained based on the following Table 1 or Table 2, in the three-sample testing step.

[Table 1]

| Case | Genotype | | Paternity Index |
| --- | --- | --- | --- |
| | Alleged Father | Fetus | PI |
| (I) | AA | AA | 1/a |
| (II) | AA | AB | kb |
| (III) | BB | AA | k0 |
| (IV) | BB | AB | 1/b |
| (V) | AB | AA | 0.5/a |
| (VI) | AB | AB | 0.5/b |

[Table 2]

| Case | Genotype | | | Paternity Index |
| --- | --- | --- | --- | --- |
| | Mother | Alleged Father | Fetus | PI |
| (I) | AA | AA | AA | 1/a |
| (II) | AA | AA | AB | kb |
| (III) | AA | BB | AA | k0 |
| (IV) | AA | BB | AB | 1/b |
| (V) | AA | AB | AA | 0.5/a |
| (VI) | AA | AB | AB | 0.5/b |

(In Tables 1 and 2, an allele having a relatively high signal intensity is denoted as A, and an allele having a relatively small signal intensity is denoted as B, in the analysis of the circulating cell-free nucleic acid samples. In Table 1, k0 is the false negative rate, kb is the false positive rate, a represents the appearance frequency of A, and b is the appearance frequency of B.)

[3] The non-invasive prenatal paternity testing method according to [1] or [2], including the three-sample testing step and an MC filter step defined below.

<MC Filter Step>

The genotyping of the mother is performed based on a sample containing a nucleic acid having genetic information of the mother and collected from the mother and substantially not containing a nucleic acid having genetic information of the fetus, and

if

(i) the polymorphic locus which is a homozygote of alleles mutually different in genotype between the mother and the fetus or circulating cell-free nucleic acid,

and/or

(ii) the polymorphic locus in which the genotype in the mother is a heterozygote and the genotype of the circulating cell-free nucleic acid is a homozygote,

are observed in a predetermined number or more, the paternity test to be performed based on the collected samples is stopped without executing.

[4] The non-invasive prenatal paternity testing method according to any one of [1] to [3], wherein the verification step defined below is executed based on samples collected from a specified test-subject family.

<Verification Step>

In order to verify an erroneous determination caused by a mix-up of a sample with a sample, which is collected from a test-subject family different from the specified test-subject family, verification is performed according to verification pattern 1 and/or verification pattern 2 defined below.

(Verification Pattern 1)

Based on the genotyping result of the fetus belonging to the specified test-subject family,

optionally, the genotyping result of the mother belonging to the specified test-subject family, and
the genotyping result of an alleged father belonging to the different test-subject family,
a paternity test is performed on a combination of the alleged father and the fetus.

(Verification Pattern 2)

Based on the genotyping result of a fetus belonging to the different test-subject family,

optionally, the genotyping result of the biological mother of the fetus, belonging to the different test-subject family, and
the genotyping result of the alleged father belonging to the specified test-subject family,
a paternity test is performed on a combination of the alleged father and the fetus.

[5] The non-invasive prenatal paternity testing method according to [4], wherein, in the verification step,
the verification is performed according to verification pattern 1 and/or verification pattern 2 with reference to databases storing the genotyping results of an alleged father, a fetus, and optionally the biological mother of the fetus, belonging to one or two or more different test-subject families.
[6] The non-invasive prenatal paternity testing method according to [5], comprising, after completion of genotyping of the alleged father, the fetus, and optionally the mother belonging to the specified test-subject family, storing results in the database and updating the database, and

executing the verification step with reference to the updated database, in the verification step,
wherein,
in the verification step, the verification according to verification pattern 1 and/or verification pattern 2 is performed by regarding the genotyping results of the specified test-subject family reflected in the database, as the genotyping results of the different test-subject family.

[7] The non-invasive prenatal paternity test according to any one of [4] to [6], wherein the genotyping of the alleged father and the genotyping of the fetus are executed

at mutually different places and/or different times, and/or
executed by mutually different operators.

At a site of a blood-relationship test service, a person in charge examines a set of samples of alleged blood relatives collected from the specified test-subject family and a sample collected from a specified person. In this case, a sample mix-up occurs and an incorrect blood-relationship test result is sometimes output. As a method of preventing such a sample mix-up, the same examination is conducted twice for a double check, but there is a problem in that the examination cost becomes double.
In view of such a problem, a second object of the invention is to provide a novel technology for verifying an erroneous determination caused by the sample mix-up in a blood-relationship test.
The invention for attaining the second object is as follows.

[1] A method of verifying an erroneous determination caused by a sample mix-up in a blood-relationship test conducted between a specified person and an alleged blood relative based on a set of samples collected from a specified test-subject family including an alleged blood relative who is a subject of a test for the presence or absence of a biological blood relationship, a specified person, and an optional biological blood relative of the specified person, the method including a verification step defined below.

<Verification Step>

In order to verify an erroneous determination caused by a mix-up of a sample collected from a different test-subject family and a sample collected from the specified test-subject family, verification is performed according to verification pattern 1 and/or verification pattern 2 defined below.

(Verification Pattern 1)

Based on the genotyping result of the specified person belonging to the specified test-subject family,

optionally, the genotyping result of a biological blood relative of the specified person belonging to the specified test-subject family, and
the genotyping result of an alleged blood relative belonging to the different test-subject family,
a blood-relationship test is performed on a combination of the alleged blood relative and the specified person.

(Verification Pattern 2)

Based on the genotyping result of a specified person belonging to the different test-subject family,

optionally, the genotyping result of a biological blood relative of the specified person belonging to the different test-subject family, and
the genotyping result of the alleged blood relative belonging to the specified test-subject family,
a blood-relationship test is performed on a combination of the alleged blood relative and the specified person.

[2] The verification method according to [1], which is a method of verifying an erroneous determination caused by a sample mix-up in a paternity test between an alleged parent and a child based on a set of samples collected from a specified test-subject family including the alleged parent who is a test subject for determining whether or not a biological parent-child relationship exists, the child, and optionally the biological parent, wherein

the alleged blood relative is the alleged parent,
the specified person is the child,
the biological relative of the specified person is the biological parent of the child, different in gender from the alleged parent, and
the blood-relationship test is a paternity test; and
the verification method includes a verification step defined below.

<Verification Step>

In order to verify an erroneous determination caused by a mix-up of a sample with a sample, which is collected from a test-subject family different from the specified test-subject family, verification is performed according to verification pattern 1 and/or verification pattern 2 defined below.

(Verification Pattern 1)

Based on

the genotyping result of the child belonging to the specified test-subject family,
optionally, the genotyping result of the biological parent belonging to the specified test-subject family, and
the genotyping result of an alleged parent belonging to a different test-subject family, a paternity test is performed on a combination of the alleged parent and the child.

(Verification Pattern 2)

Based on the genotyping result of a child belonging to a different test-subject family,

optionally, the genotyping result of the biological parent of the child, belonging to a different test-subject family,
the genotyping result of the alleged parent belonging to the specified test-subject family,
a paternity test is performed on a combination of the alleged parent and the child.

[3] The verification method according to [1] or [2], wherein the blood-relationship test includes a test to be performed based on the genotyping results of two persons: the alleged blood relative and the specified person, who are test subjects for the presence or absence of a biological blood relationship.
[4] The verification method according to any one of [1] to [3], wherein the blood-relationship test includes a test to be performed based on the genotyping result of three persons: the alleged blood relative, who is a test subject for determining whether or not a biological parent-child relationship exists, the specified person, and the biological blood relative.

[5] The verification method according to any one of [1] to [4], wherein, in the verification step,

verification is performed according to verification pattern 1 and/or verification pattern 2 with reference to a database storing genotyping results of an alleged blood relative and a specified person belonging to one or two or more of the different test-subject families.

[6] The verification method according to [2], wherein, in the verification step,

verification is performed according to verification pattern 1 and/or verification pattern 2 with reference to a database storing genotyping results of an alleged parent, a child, and optionally the biological parent of the child belonging to one or two or more of the different test-subject families.

[7] The verification method according to [5], including, after completion of the genotyping of the alleged blood relative belonging to the specified test-subject family and the specified person, storing the results thereof in the database and updating the database.

[8] The verification method according to [6], including, after completion of the genotyping of the alleged parent belonging to the specified test-subject family, the child, optionally the biological parent, storing the results in the database and updating the database.

[9] The verification method according to [7] or [8], wherein

the verification step includes

executing the verification step with reference to the updated database, and performing verification according to verification pattern 1 and/or verification pattern 2 by regarding the genotyping results of the specified test-subject family reflected in the database, as the genotyping results of the different test-subject family.

[10] The verification method according to any one of [1] to [9], wherein

the genotyping of the alleged blood relative and the genotyping of the specified person are executed at mutually different places and/or different times, and/or
executed by mutually different operators.

Advantageous Effects of Invention

According to the invention for attaining the first object, it is possible to simply perform a non-invasive prenatal paternity test using a clear criterion such as criterion a or criterion b based on the genotyping results of two persons: an alleged father and a fetus.

In addition, according to the invention for attaining the second object, it is possible to verify an erroneous determination caused by a sample mix-up, with a high degree of accuracy.

Brief Description of Drawings

Fig. 1 is a flowchart of an embodiment including a two-sample testing step and a verification step.
Fig. 2 is a flowchart of an embodiment including an MC filter step, a three-sample testing step, and a verification step.
Fig. 3 illustrates a schematic diagram of a verification pattern 1 for performing a two-sample test with reference to a database.
Fig. 4 illustrates a schematic diagram of a verification pattern 2 for performing a two-sample test with reference to a database.
Fig. 5 illustrates a schematic diagram of a verification pattern 1 for performing a three-sample test with reference to a database.
Fig. 6 illustrates a schematic diagram of a verification pattern 2 for performing a three-sample test with reference to a database.
Fig. 7 illustrates a graph obtained by analyzing circulating cell-free nucleic acid samples and plotting signal intensities of alleles per polymorphic locus. The vertical axis represents the ratio of the signal indicating the presence of a specified allele with respect to the combined signal intensities derived from a specified polymorphic locus.

Description of Embodiments

<1> Non-invasive Prenatal Paternity Testing Method

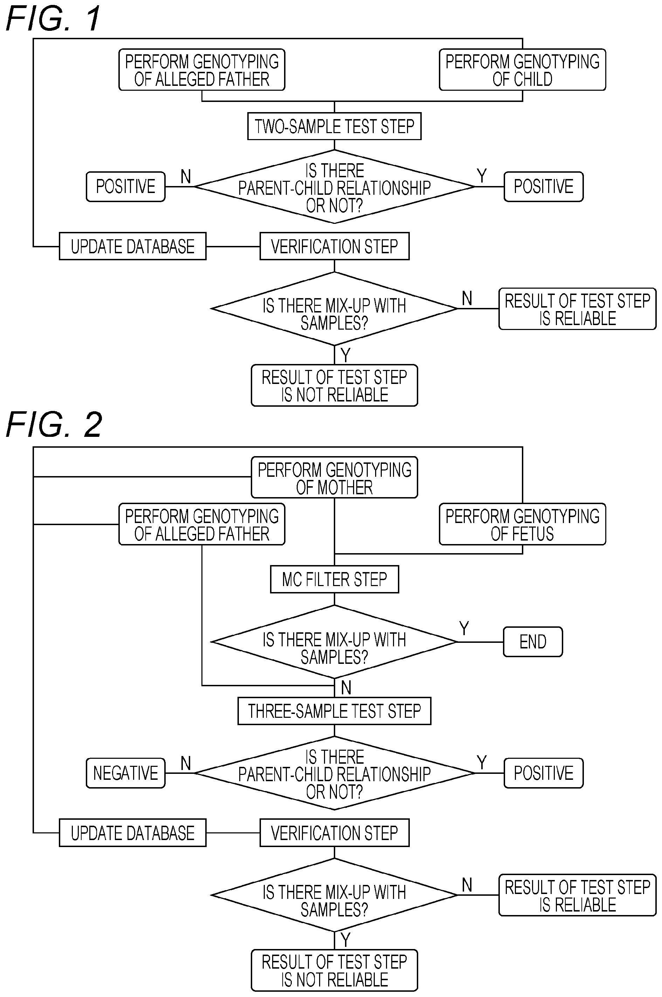

The invention for attaining the first object relates to a non-invasive prenatal paternity testing method using maternal blood (circulating cell-free nucleic acid sample). An embodiment of the non-invasive prenatal paternity testing method of the invention will be described appropriately with reference to Figs. 1 and 2. Note that the invention is not limited to the embodiments shown in Figs. 1 and 2.

The invention includes two-sample testing steps and/or three-sample testing steps defined below.

<Two-sample Testing Step>

The two-sample testing step is a step of determining whether or not a biological parent-child relationship exists between an alleged father and a fetus based on the genotyping results of two persons: the alleged father and the fetus, at a plurality of polymorphic loci. In the two-sample testing step, the genotyping of the fetus is performed according to the determination method based on criterion a defined below.

(Determination Method Based on Criterion a)

Provided that

the allele which is contained in data obtained by analysis of a circulating cell-free nucleic acid sample collected from a pregnant mother, derived from a specified polymorphic locus and detected with the strongest signal intensity, is denoted as allele A, and
the allele detected with the same signal intensity as that of allele A or the second strongest signal intensity is denoted as allele B,
determination is performed using the ratio of the signal intensity (Fa) of allele A and the ratio of the signal intensity (Fb) of allele B with respect to the combined intensity of signals derived from the specified polymorphic locus, as indexes, and according to the following criterion a.

<Criterion a>

· If $0\% \leq Fb \{\leq, <\} L\%$ is satisfied, the genotype at the specified polymorphic locus of a fetus is determined to be AA homozygote
· If $M\% \{\leq, <\} Fb \{\leq, <\} N\%$ is satisfied, the genotype at the specified polymorphic locus of a fetus is determined to be AB heterozygote
· If Fa = Fb is satisfied, the genotyping of the specified polymorphic locus is skipped without being performed
· If Fb does not satisfy any of the above conditions, the genotyping of the specified polymorphic locus is skipped without being performed.

N is a numerical value of less than 50. Specific numerical values of L, M, and N can be appropriately set within a range satisfying a relational expression of $0 \{\leq, <\} L < M \{\leq, <\} N$. Note that the notation of $\{\leq, <\}$ indicates that either one of the inequality signs, $\leq$ and $<$, can be arbitrarily selected.

The lower limit value of L can be set to, for example, 0.1 or more. The upper limit value of L can be set to, for example, less than 1, 0.8 or less, or 0.5 or less.

The lower limit value of M can be set to, for example, 0.5 or more, 0.8 or more, or 1 or more. The upper limit value of M can be set to, for example, 3 or less, or 2 or less.

From the viewpoint of preventing mistakenly identifying the signal of the maternally derived allele for that of the fetus-derived allele, N is preferably set to 40 or less, more preferably 30 or less, and still more preferably 25 or less. Owing to the setting, the polymorphic locus at which a signal from the allele derived from a mother is highly possibly misidentified as that from the allele derived from a fetus can be eliminated from the base
of a paternity test, with the result that the possibility of erroneous determination can be suppressed.

If noise derived from a specified polymorphic locus exceeds a predetermined standard, the condition that the genotyping of the specified polymorphic locus is skipped without being performed may be added to the above-described criterion a. That is, provided that alleles which are contained in data obtained by analysis of a circulating cell-free nucleic acid sample, derived from a specified polymorphic locus, and detected with the third and fourth strongest signal intensities, are denoted as allele C and allele D, respectively, the following condition for skipping may be added to the above-described criterion a using the ratio (Fc) of the signal intensity of allele C and the ratio (Fd) of the signal intensity of allele D with respect to the combined signal intensities derived from the specified polymorphic locus, as indexes.

· If K {≤, <} Fc + Fd is satisfied, the genotyping of the specified polymorphic locus is skipped without being performed.

The numerical value of K can be arbitrarily set. For example, an arbitrary numerical value of 10 or less, and preferably 5 or less, can be employed.

<Three-sample Testing Step>

The three-sample testing step is a step of determining whether or not a biological parent-child relationship exists between an alleged father and a fetus based on the genotyping results of three persons: the biological mother, the alleged father, and the fetus at a plurality of polymorphic loci. In the three-sample testing step, the genotyping of the fetus is performed according to the determination method based on criterion a and/or the determination method based on criterion b, defined below.

(Determination Method Based on Criterion b)

Provided that

the allele which is contained in data obtained by analysis of a circulating cell-free nucleic acid sample, derived from a specified polymorphic locus determined as a homozygote in the mother, and detected with the strongest signal intensity, is denoted as allele A, and
the allele which is detected with the same signal intensity as allele A or the second strongest signal intensity is denoted as allele B,
determination is performed using the ratio of the signal intensity (Fa) of allele A and the ratio of the signal intensity (Fb) of allele B with respect to the combined intensity of signals derived from the specified polymorphic locus, as indexes, and according to the following criterion b.

<Criterion b>

· If 0% ≤ Fb {≤, <} L% is satisfied, the genotype at the specified polymorphic locus of a fetus is determined to be AA homozygote
· if M% {≤, <} Fb is satisfied, then the genotype at the specified polymorphic locus of the fetus is determined to be AB heterozygote
· If Fa = Fb is satisfied, the genotyping of the specified polymorphic locus is skipped without being performed
· If Fb does not satisfy any of the above conditions, the genotyping of the specified polymorphic locus is skipped without being performed

(is satisfied.)

Specific numerical values of L and M can be appropriately set within a range satisfying the relational expression of 0 {≤, <} L < M. Note that the notation of {≤, <} indicates that either one of the inequality signs, ≤ and <, can be arbitrarily selected.
The lower limit value of L can be set to, for example, 0.1 or more. The upper limit value of L can be set to, for example, less than 1, 0.8 or less, or 0.5 or less.
The lower limit value of M can be set to, for example, 0.5 or more, 0.8 or more, or 1 or more. The upper limit value of M is not limited.
If noise derived from a specified polymorphic locus exceeds a predetermined standard, the condition of skipping the genotyping of the specified polymorphic locus without performing it, may be added to the above-described criterion b. That is, provided that alleles which are contained in data obtained by analysis of a circulating cell-free nucleic acid sample, derived from a specified polymorphic locus, detected with the third and fourth strongest signal intensities, are denoted as allele C and allele D, respectively, the following condition for skipping may be added to the above-described criterion b using the ratio (Fc) of the signal intensity of allele C and the ratio (Fd) of the signal intensity of allele D to the combined signal intensities derived from the specified polymorphic locus, as indexes.

· If K {≤, <} Fc + Fd is satisfied, the genotyping of the specified polymorphic locus is skipped without being performed.

The numerical value of K can be arbitrarily set. For example, an arbitrary numerical value of 10 or less, and preferably 5 or less, can be employed.

Note that, in the invention, means for analyzing a nucleic acid sample for genotyping is not particularly limited. For example, an analysis method that can separately detect single-base substitutions (SNVs) at a polymorphic locus is preferable. Examples thereof include base sequence analysis, mass spectrometry, digital PCR, SNV microarray, and real-time PCR. Examples of the means for the base sequence analysis include a next-generation sequencer (NGS).

As the polymorphic locus to be analyzed, a single-base polymorphic locus can be preferable.

The genotyping of an alleged father and the genotyping of a mother in a case of the three-sample testing step can be easily performed according to a conventional method. For example, genotyping can be easily performed by analyzing a sample usually used in a genetic test, such as an intraoral mucosal cell sample collected from the oral cavity.

In a case of performing the two-sample testing step, whether or not a biological parent-child relationship exists between a fetus and an alleged father is analyzed based on the genotyping result of the fetus and the genotyping result of the alleged father determined by the above-described method (Fig. 1).

In a case of performing the three-sample testing step, whether or not a biological parent-child relationship exists between a fetus and an alleged father is analyzed based on the genotyping result of the fetus, the genotyping result of the alleged father, and the genotyping result of the biological mother of the fetus determined by the above-described method (Figs. 1 and 2).

The methods for the paternity test in the two-sample testing step and the three-sample testing step are not particularly limited. In a mode where the genotyping of a single-base polymorphic locus is performed, the paternity index (PI) can be determined for each single-base polymorphic locus based on the genotyping results of an alleged father and a fetus. The method of calculating the paternity index is not particularly limited. In the case of the two-sample testing step, calculation can be made, according to, for example, the following Table 1, while in the case of the three-sample testing step, for example, calculation can be made according to the following Table 1 or Table 2.

[Table 1]

| Case | Genotype | | Paternity Index |
|------|----------|-------|-----------------|
|      | Alleged Father | Fetus | PI |
| (I)   | AA | AA | 1/a |
| (II)  | AA | AB | kb |
| (III) | BB | AA | k0 |
| (IV)  | BB | AB | 1/b |
| (V)   | AB | AA | 0.5/a |
| (VI)  | AB | AB | 0.5/b |

[Table 2]

| Case | Genotype | | | Paternity Index |
|------|--------|----------------|-------|-----------------|
|      | Mother | Alleged Father | Fetus | PI |
| (I)   | AA | AA | AA | 1/a |
| (II)  | AA | AA | AB | kb |
| (III) | AA | BB | AA | k0 |
| (IV)  | AA | BB | AB | 1/b |
| (V)   | AA | AB | AA | 0.5/a |
| (VI)  | AA | AB | AB | 0.5/b |

In Tables 1 and 2, an allele having a relatively high signal intensity is denoted as A, and an allele having a relatively small signal intensity is denoted as B, in the analysis of the circulating cell-free nucleic acid samples. In Table 1, k0 is the false negative rate, kb is the false positive rate, a is the appearance frequency of A, and b is the appearance frequency of B.

Note that k0 and kb, which can be appropriately set depending on the accuracy of the examination system, can be, for example, 0.1 or less, preferably 0.01 or less, and more preferably 0.001 or less.

After the PI is determined for each single-base polymorphic locus, the combined paternity index (CPI) may be obtained. CPI is obtained as a combined product of PIs.

The invention, it is preferable, as an embodiment, that "positive", "negative", or "undeterminable" is determined based on a criterion determined in advance according to the numerical value of CPI, for example, as shown below.

$10^4 \leq$ CPI ... Result: "positive"
$10^{-6} \leq$ CPI $< 10^4$... Result: "undeterminable"
CPI $<10^{-6}$ ... Results: "negative"

The criteria described above are examples. Specific reference values of the CPIs for determination can be appropriately set.

After the CPI is calculated, the probability of paternity (POP) may be obtained. POP can be calculated using the following formula. Note that P in the formula represents the prior probability of paternity.

[Mathematical formula 1]

$$POP = \frac{CPI \times P}{CPI \times P + (1 - P)}$$

In an embodiment including a three-sample testing step, an MC filter step defined below is preferably included. (Fig. 2).

<MC Filter Step>

The genotyping of the mother is performed based on a sample containing a nucleic acid having genetic information of the mother and collected from the mother and substantially not containing a nucleic acid having genetic information of the fetus. If the following (i) and/or (ii) are observed in a predetermined number, the paternity test to be performed based on the collected samples is stopped without executing.

(i) The polymorphic locus which is a homozygote of alleles mutually different in genotype between the mother and the fetus or circulating cell-free nucleic acid,
(ii) The polymorphic locus in which the genotype in the mother is a heterozygote and the genotype of the circulating cell-free nucleic acid is a homozygote.

The circulating cell-free nucleic acid sample contains a nucleic acid derived from a mother as the major nucleic acid. Thus, as a result of performing the genotyping of a fetus or a circulating cell-free nucleic acid by analyzing the circulating cell-free nucleic acid sample, it is usually unthinkable that the genotype of the mother and the fetus or circulating cell-free nucleic acid falls under the conditions described in (i) and/or (ii) above. In such a case, a sample mix-up is suspected.

If a predetermined number or more of cases in which such a sample mix-up is suspected are observed, the MC filter step is performed in order to stop the paternity test without executing the three-sample testing step (Fig. 2), and prevent the occurrence of an erroneous determination of the test result and unnecessary work.

The "predetermined number" can be set arbitrarily.

The predetermined number can be set to a number which is less than 5%, preferably less than 3%, and more preferably less than 1% of the total number of polymorphic loci observed in genotyping.

The specific number as the predetermined number, is not particularly limited, but can be set to 5 or less, more preferably 3 or less, and still more preferably 1.

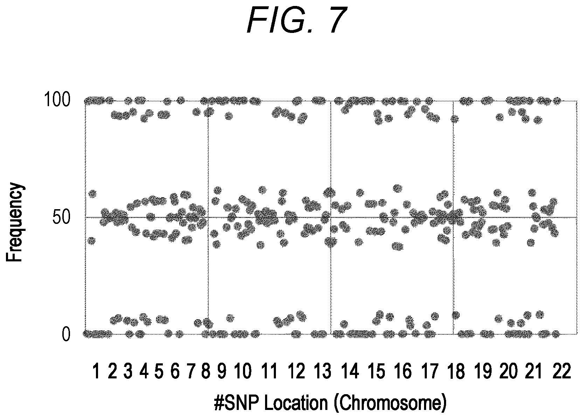

The genotyping of a circulating cell-free nucleic acid for identifying the genotype of the circulating cell-free nucleic acid will be described with reference to Fig. 7.

Circulating cell-free nucleic acid samples contain major nucleic acids derived from a mother and minor nucleic acids derived from a fetus. Thus, if circulating cell-free nucleic acid samples are analyzed and the ratio of the signal indicating the presence of a predetermined allele to the combined signal intensities derived from a specified polymorphic locus is plotted, the signal is distributed in a range of 0 to 25%, 35 to 65%, and 75 to 100%, as shown in Figure 7. On the assumption that there is no contamination with DNA of another person from the outside, and a case in which the above-described ratio at each polymorphic locus is 5% or more is treated as a true signal.

In consideration of this, provided that

the allele which is contained in data obtained by analysis of a circulating cell-free nucleic acid sample, derived from a specified polymorphic locus, and detected with the strongest signal intensity, is denoted as allele A, and the allele detected with the same signal intensity as that of allele A or the second strongest signal intensity is denoted as allele B,

the genotyping of the circulating cell-free nucleic acid is performed according to the following criterion "c" using the ratio of the signal intensity ($F_a$) of allele A and the ratio of the signal intensity ($F_b$) of allele B with respect to the combined intensity of signals derived from the specified polymorphic locus, as indexes.

<Criterion c>

· If $X \{\leq, <\} F_a \{\leq, <\} Y\%$ is satisfied, the genotype at the specified polymorphic locus of the circulating cell-free nucleic acid is determined to be AB heterozygote.

· If $Z\% \{\leq, <\} F_a \leq 100$ is satisfied, the genotype at the specified polymorphic locus of the circulating cell-free nucleic acid is determined to be AA homozygote.

· If $F_a = F_b$ is satisfied, the genotype of the specified polymorphic locus is determined to be AB heterozygote.

Specific numerical values of X, Y, and Z can be appropriately set within a range satisfying a relational expression of $X \{\leq, <\} Y < Z$. Note that the notation $\{\leq, <\}$ indicates that either one of the inequality signs of $\leq$ and $<$ can be arbitrarily selected.

Note that, if the ratio does not correspond to any one of the above cases, an embodiment in which the genotyping of the specified allele is skipped without being performed may be employed.

The numerical value of X can be set to an arbitrary numerical value of preferably 40 or more, and more preferably 45 or more.

The numerical value of Y can be set to an arbitrary numerical value of preferably 65 or less, and more preferably 60 or less.

The numerical value of Z can be set to an arbitrary numerical value of preferably 75 or more, and more preferably 95 or more.

In an embodiment of the invention, after the above-described "the genotyping of a mother based on a sample containing a nucleic acid having genetic information of the mother collected from the mother and substantially not containing a nucleic acid having genetic information of a fetus" and "the genotyping of a circulating cell-free nucleic acid based on the circulating cell-free nucleic acid sample collected from the mother" are performed, the MC filter step is executed.

The stop condition employed in the MC filter step may be either one or both of (i) and (ii) described above.

Note that even if the three-sample testing step is performed, the MC filter step is not essential. The MC filter step is an optional step, and may or may not be performed.

In an embodiment of the invention, if the stop decision is not made in the MC filter step, the test based on the collected sample is continued (Fig. 2). In contrast, when the stop decision is made in the MC filter step, the three-sample testing step based on the collected samples is stopped without executing (Fig. 2).

In the case of a standard legal test, it is necessary for a witness to attend in all cases of collecting a sample. However, when the MC filter step is performed, it is possible to determine whether a nucleic acid sample of a mother is mixed up with a circulating cell-free nucleic acid sample. Because of this, the attendance of a witness, which is required for a legal test at the time of collecting maternal blood, is no longer required, providing an advantage: the legal test can be performed only by attendance at the time of collecting a Genomic DNA sample of the mother.

The invention may have a mode including either one or both of the two-sample testing step and the three-sample testing step. In the mode where both the two-sample testing step and the three-sample testing step are included, a mode where either one of the test results may be output as a final test result, is acceptable, or a mode including a step of comparing the test results of the two testing steps, is acceptable. In addition, an embodiment in which a final test result is output according to a predetermined criterion based on the pattern as to whether two test results match or not, is acceptable.

The predetermined criterion described above can be arbitrarily set. For example, the criteria according to the combination patterns 1 to 9, which are combinations of the test results of the two-sample testing step and the three-sample testing step shown in the following Table 3, may be employed.

[Table 3]

| Pattern | Test result by two-sample testing step | Test result by three-sample testing step | Final paternity test result |
|---|---|---|---|
| 1 | Positive | Positive | Positive |
| 2 | Positive | Negative | Undeterminable |
| 3 | Positive | Undeterminable | Positive |
| 4 | Negative | Positive | Undeterminable |
| 5 | Negative | Negative | Negative |
| 6 | Negative | Undeterminable | Negative |
| 7 | Undeterminable | Positive | Positive |
| 8 | Undeterminable | Negative | Negative |
| 9 | Undeterminable | Undeterminable | Undeterminable |

The non-invasive prenatal paternity testing method of the invention may further include a verification step for verifying an erroneous determination caused by a sample mix-up (Figs. 1 and 2).

Figs. 1 and 2 illustrate embodiments in which a verification step is executed regardless of the result of a two-sample testing step or a three-sample testing step. In the case of such an embodiment, it is possible to verify whether an erroneous determination caused by a sample mix-up is contained in a negative or positive result of the biological parent-child relationship in the two-sample testing step or the three-sample testing step.

If it was determined in the verification step that samples were mixed up, it can be determined that the result of the two-sample testing step or the three-sample testing step is not reliable.

Figs. 1 and 2 show merely embodiments. It is possible to employ an embodiment in which the verification step is executed depending on the result of the two-sample testing step or the three-sample testing step.

In a case in which two or more operators each independently perform the examination, and in a case in which a single operator performs the examination a plurality of times in different places/times, the possibility that the same sample mix-up event occurs in all examinations is extremely low.

It is acceptable to employ an embodiment in which the verification step is performed if the "negative" determination result is obtained in each examination under such a double-check system.

In contrast, it is also acceptable to employ an embodiment in which if a "positive" determination result is obtained in each examination under the double-check system, the determination result is assumed correct, and the verification step is not performed. Of course, a triple-check system in which further a verification step is performed may be employed.

A specific embodiment of the verification step will be described in detail in the following section Verification Method in the description of the invention.

<2> Verification Method

The invention relates to a method of verifying an erroneous determination of a blood-relationship test caused by the mix-up of a set of samples collected from a specified test-subject family.

The verification method of the invention can be applied to a method of testing biological parent-child relationship, a biological sibling relationship, a biological relationship between a grandparent and a grandchild, and all other biological blood relationships.

The test-subject family herein refers to a group including at least a specified person and an alleged blood relative thereof, who are test subjects for determining whether or not they have a biological blood relationship. In the test-subject family, one or two or more biological relatives of the specified person may be included, optionally.

That is, it is possible to employ a mode in which only two persons: an alleged blood relative and a specified person, are included in the test-subject family, and a mode in which three persons: an alleged blood relative, the specified person, and a biological blood relative of the specified person, are included in the family. In other words, the verification method of the invention can be applied to both the above-described two-sample testing step and the three-sample testing step.

For example, in a case in which a biological sibling relationship is tested, a test-subject family is constituted of a specified person and an alleged sibling of the specified person. In this case, a biological relative, such as a biological parent or sibling of the specified person, may optionally be included in the test-subject family.

In a case of testing the relationship between biological grandparents and a grandchild, the alleged grandfather or the alleged grandmother and the grandchild constitute a test-subject family. In this case, optionally, biological blood relatives, such as a biological parent of the grandchild, a biological grandmother or a grandfather different in gender from an alleged grandfather or alleged grandmother who is an alleged blood relative, may be included in the test-subject family.

In a case in which a paternity test is performed, an alleged parent and a child, who are test subjects of determining whether or not a biological parent-child relationship exists, are included in the test-subject family. The test-subject family may optionally include a biological parent of the child different in gender from the alleged parent.

That is, the test-subject family may have a mode in which only two persons: an alleged parent and a child, are included, and a mode in which three persons: an alleged parent, a child, and a biological parent, are included. In other words, the verification method of the invention can be applied to both the above-described two-sample testing step and the three-sample testing step.

The "biological parent of the child different in gender from the alleged parent", is a "biological mother of the child" if the alleged parent is an "alleged father", and is a "biological father of the child" if the alleged parent is an "alleged mother".

That is, the verification method of the invention can be applied to both the father-child test and the mother-child test.

Note that it is possible to employ an embodiment including a parent (father or mother) as a specified person constituting the test-subject family, and an alleged child as an alleged blood relative.

Hereinafter, the description will be made focusing on an embodiment in which the verification method of the invention is applied to a paternity test in a case in which a "child", an "alleged father", and optionally a "biological mother" are included as a test-subject family. As a matter of course, it should be noted that the invention is not limited to the paternity test described below and the invention can be applied to the verification of all blood relationship tests.

The verification method of the invention can also be applied to the paternity test performed between a child after birth and an alleged parent. Furthermore, it is natural to apply the verification method to the paternity test performed between a prenatal child (fetus) and an alleged parent (alleged father), such as a non-invasive prenatal paternity testing method of the invention described above.

When the verification method of the invention is applied to a father-child test, the specific manner of the father-child test is not limited. For example, PI calculation may be performed based on Table 1 or Table 2 described above. Note that, in the case of a child after birth, "fetus" in Table 1 or Table 2 described above can be rephrased as "child after birth" and applied as it is. Further, the CPI may be calculated based on the calculated PI, or POP may be obtained based on the CPI.

If the verification method of the invention is applied to a mother-child test, the specific manner of the mother-child test is not limited. For example, the maternity index (MI) can be calculated according to PI calculation in the father-child test. Furthermore, the combined maternity Index (CMI) may be calculated according to the CPI calculation method based on the calculated MI. Moreover, the probability of maternity (POM) may be obtained according to the POP calculation method based on the calculated CMI.

As a matter of course, the verification method of the invention can also be applied to a paternity test not employing the formula of PI or MI shown in Table 1 or Table 2. For example, other than the method using the formula of Table 1 or Table 2, the verification method of the invention can be applied to a method using the likelihood ratio representing the probability of the parent-child relationship.

In a case in which the genotyping of an alleged blood relative and the genotyping of a specified person are performed by a single operator at the same place and the same time, there is a possibility that samples of not a part of the test-subject family members but all samples of the test-subject family members are mixed up. In such cases, if all samples of the test-subject family members were mixed up, the event of the sample mix-up cannot definitively be detected in the verification step described later.

In order to solve this problem, in a preferred embodiment of the verification method of the invention, the genotyping of an alleged blood relative and the genotyping of a specified person are executed at mutually different places and/or different times, and/or executed by mutually different operators.

In the case of a paternity test, the genotyping of an alleged parent and the genotyping of a child are executed at mutually different places and/or different times, and/or executed by mutually different operators.

As described above, in a case in which genotyping analysis of an alleged blood relative and a specified person is performed at different places/different times/by different operators, an effect of reducing the likelihood of occurring a similar sample mix-up event in genotyping analyses can be obtained. If such an embodiment of the genotyping is employed and the verification is executed according to the verification step described later, an extremely advantageous effect of significantly improving the verification accuracy can be obtained.

The verification method of the invention is characterized by including a verification step defined below.

<Verification Step>

In order to verify an erroneous determination caused by a mix-up of a sample with a sample, which is collected from a test-subject family different from the specified test-subject family, verification is performed according to verification pattern 1 and/or verification pattern 2 defined below.

(Verification Pattern 1)

Based on

the genotyping result of the child belonging to the specified test-subject family,
optionally, the genotyping result of the biological parent belonging to the specified test-subject family, and
the genotyping result of an alleged parent belonging to a different test-subject family, a paternity test is performed on a combination of the alleged parent and the child.

(Verification Pattern 2)

Based on the genotyping result of a child belonging to a different test-subject family,

optionally, the genotyping result of the biological parent of the child, belonging to a different test-subject family,
the genotyping result of the alleged parent belonging to the specified test-subject family,
a paternity test is performed on a combination of the alleged parent and the child.

Verification pattern 1 is a pattern of a paternity test performed on a combination of a child belonging to a specified test-subject family and an alleged parent belonging to a different test-subject family.

The paternity test performed herein may be a two-sample test or a three-sample test.

The two-sample test in verification pattern 1 is a paternity test performed based on the genotyping results of two persons: a child belonging to a specified test-subject family and an alleged parent belonging to a different test-subject family. To a specific manner thereof, the above description regarding the two-sample testing step can be applied as it is.

The three-sample test in verification pattern 1 is a paternity test performed based on the genotyping results of three persons: a child belonging to a specified test-subject family, a biological parent of the child belonging to the specified test-subject family, and an alleged parent belonging to a different test-subject family. To a specific manner thereof, the above description regarding the three-sample testing step can be applied as it is.

Verification pattern 2 is a pattern of a paternity test performed on a combination of a child belonging to a different test-subject family and an alleged parent belonging to a specified test-subject family.

The paternity test performed herein may be a two-sample test or a three-sample test.

The two-sample test in verification pattern 2 is a paternity test performed based on the genotyping results of two persons: a child belonging to a different test-subject family and an alleged parent belonging to a specified test-subject family. To a specific manner thereof, the above description regarding the two-sample testing step can be applied as it is.

The three-sample test in verification pattern 2 is a paternity test performed based on the genotyping results of three persons: a child belonging to a different test-subject family, a biological parent of the child belonging to a different test-subject family, and an alleged parent belonging to a specified test-subject family. To a specific manner thereof, the above description regarding the three-sample testing step can be applied as it is.

Note that, if the paternity test, which is a subject for verifying whether or not samples are mixed up, was a two-sample test, the paternity test performed in the verification step is not limited to the two-sample test, and no problem is produced even if the three-sample test is performed in the verification step.

In contrast, if the paternity test, which is a subject for verifying whether or not samples are mixed up, was a three-sample test, the paternity test performed in the verification step is not limited to the three-sample test, and no problem is produced even if the two-sample test is performed in the verification step.

In verification pattern 1 and/or verification pattern 2, if a test result shows that a biological parent-child relationship is positive, it can be determined that the possibility of mix-up of a sample from a specified test-subject family with a sample from a different test-subject family is high. That is, the possibility that samples of subjects belonging to a specific test-subject family and having a biological parent-child relationship were treated as samples belonging to a different test-subject family, is high, and it can be determined that the result of the paternity test as a verification subject, contains an erroneous determination.

In addition, if the "negative" determination of the paternity test as a verification subject, is a correct result without any sample mix-up, it can be confirmed, in the verification step, that the determination result was not wrong. Furthermore, even if the "negative" determination of the paternity test as a verification subject, is caused by a sample mix-up, if a positive result on a biological parent-child relationship was not obtained in the verification step, the determination result of the paternity test as a verification subject, becomes "negative".

That is, if a test result that denies the biological parent-child relationship was obtained in the paternity test as a verification subject, and a positive biological parent-child relationship result was not obtained also in the verification step, the existence of the biological parent-child relationship, which is a verification subject, between the alleged parent and child can be denied.

If it is determined that the result of the paternity test as a verification subject, contains an erroneous determination caused by a mix-up of a sample with a sample, which is collected from a test-subject family different from a specified test-subject family, by way of a verification step, the final conclusion: "undeterminable", may be offered. Alternatively, a sample may be collected again from a subject belonging to the specified test-subject family and tested again.

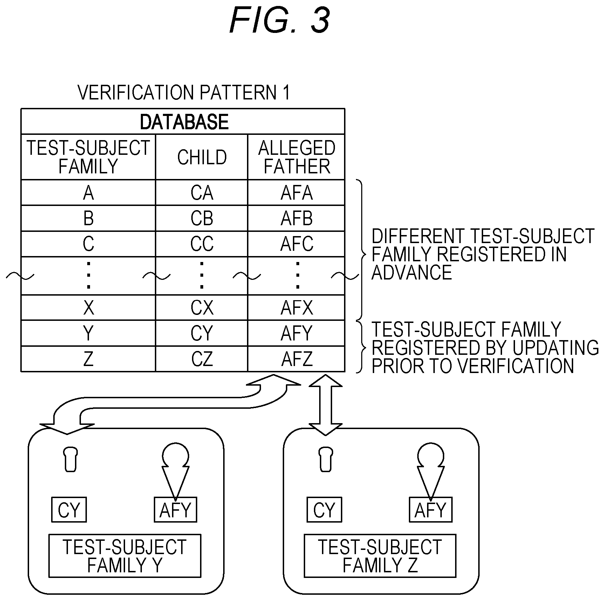

Next, an embodiment in which the verification step is executed with reference to a database storing the genotyping result of a different test-subject family will be appropriately described with reference to Figs. 3 to 6.

First, an embodiment of executing verification pattern 1 is shown in Fig. 3. In this embodiment, the paternity test (father-child test) performed on two sets of test-subject families, that is, a specified test-subject family Y including two persons: child CY and an alleged father AFY, and a test-subject family Z including two persons: a child CZ and an alleged father AFZ, is the verification subject in the verification step.

As a matter of course, the paternity test performed on one set of test-subject families may be used as the verification subject in the verification step. As shown in Fig. 3, the paternity test performed on two or more sets of test-subject families can be used as the verification subject in the verification step.

In the database, genotyping results of children CA to CX and alleged fathers AFA to AFX belonging to a plurality of sets of different test-subject families A to X, which are different from the specified test-subject families Y and Z, are stored in advance (Fig. 3).

After completion of the genotyping of child CY and alleged father AY belonging to test-subject family Y and the genotyping of child CZ and alleged father AZ belonging to test-subject family Z, the database is updated before execution of the verification step, and the genotyping results of child CY and alleged father AFY and the genotyping results of child CZ and alleged father AFZ are stored in the database (Fig. 3).

That is, the verification step is performed with reference to the database storing the genotyping results of child CA to CZ and alleged father AFA to AFZ.

In the embodiment of Fig. 3, the paternity test is performed based on each of the genotyping results of child CY and alleged fathers AFA to AFZ stored in the database. That is, the paternity test is performed for each of the combinations of child CY and alleged father AFA, child CY and alleged father AFB ...child CY and alleged father AFX, child CY and alleged father AFY, and child CY and alleged father AFZ.

In the embodiment of Fig. 3, independently of this, a paternity test is similarly performed based on each of the genotyping results of child CZ and alleged fathers AFA to AFZ stored in the database.

Hereinafter, in order to avoid duplicate description, the description will be made focusing only on a case in which a paternity test is performed based on each of the genotyping results of child CZ and alleged fathers AFA to AFZ. Needless to say, the following description also applies to a case in which a paternity test is performed based on each of the genotyping results of child CY and alleged fathers AFA to AFZ.

If the biological parent-child relationship between child CZ and any one of alleged fathers AFA to AFY was determined to be positive, it can be determined that the combination of samples of child CZ and alleged father AFZ treated as being taken from a specified test-subject family Z was mixed up with a sample taken from a person belonging to a different test-subject family.

That is, it can be determined that the test results of the paternity test executed by analysis of the samples of child CZ and alleged father AFZ belonging to test-subject family Z contain an error.

Furthermore, if a "positive" determination result has been obtained in the paternity test as a verification subject, a "positive" result should be obtained even in the verification for alleged father AFZ stored in the database by updating. In contrast, unless there is a special circumstance in which alleged father AFZ has been registered in the database as any one of alleged fathers AFA to AFY for the reason that, for example, alleged father AFZ was a subject to a paternity test in a different case, the "negative" result should be obtained in the verification on alleged fathers AFA to AFY stored in the database.

That is, if a "positive" determination result has been obtained in the paternity test as a verification subject, alleged father AFZ stored in the database by updating serves as a positive control, in the verification step, whereas alleged fathers AFA to AFY serve as a negative control unless the above-described special circumstance is present, so that it can be confirmed that the verification step itself is properly executed.

Conversely, if a "negative" determination result has been obtained in the paternity test as a verification subject, the "negative" result should be obtained even in the verification for alleged father AFZ stored in the database by updating.

That is, in the verification step, alleged father AFZ stored in the database by updating serves as a negative control, so that it can be confirmed that the verification step itself is properly executed.

Note that Fig. 3 shows an embodiment in which the database is updated based on the genotyping results of child CY and alleged father AFY belonging to test-subject family Y, and child CZ and alleged father AFZ belonging to test-subject family Z, prior to verification. However, needless to say, valid verification can be performed even if the database storing the genotyping results of only children CA to CX and alleged fathers AFA to AFX in advance, is used without updating the

EP 4 779 030 A1

database.

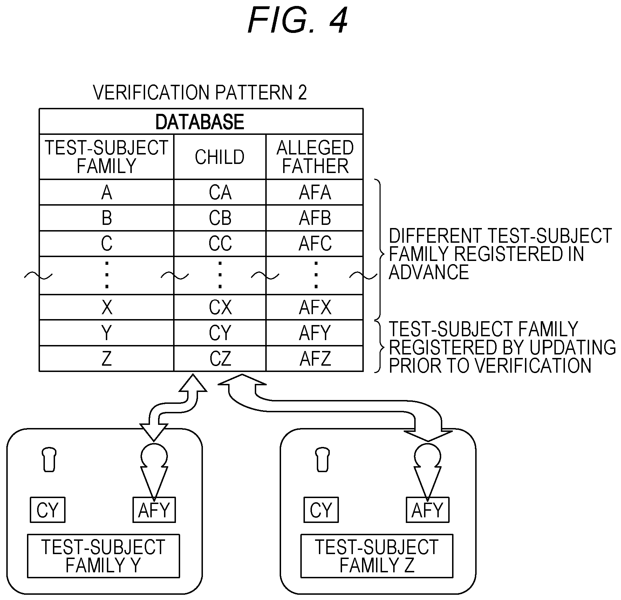

Next, an embodiment of executing verification pattern 2 is shown in Fig. 4.

The database structure of the embodiment of Fig. 4 is the same as that of Fig. 3. In the embodiment of Fig. 4, the paternity test is performed based on the genotyping result of alleged father AFY, and the genotyping results of children CA to CZ stored in the database. That is, the paternity test is performed for each of the combinations of alleged father AFY and child CA, alleged father AFY and the child CB ... alleged father AFY and the child CX, alleged father AFY and child CY, and alleged father AFY and child CZ.

In the embodiment of Fig. 4, independently of this, the paternity test is similarly performed based on the genotyping result of alleged father AFZ, and the genotyping result of each of children CA to CZ stored in the database.

Hereinafter, in order to avoid duplicate description, the description will be made focusing only on a case in which a paternity test is performed based on each of the genotyping results of alleged father AFZ and children CA to CZ. Needless to say, the following description applies to a case in which a paternity test is performed based on each of the genotyping results of alleged father AFY and children CA to CZ.

If the biological parent-child relationship between alleged father AFZ and any one of children CA to CY was determined to be positive, it can be determined that the combination of samples of child CZ and alleged father AFZ treated as being taken from a specified test-subject family Z was mixed up with a sample taken from a person belonging to a different test-subject family.

That is, it can be determined that the test results of the paternity test executed by analysis of the samples of child CZ and alleged father AFZ belonging to test-subject family Z contain an error.

Furthermore, if a "positive" determination result has been obtained in the paternity test as a verification subject, a "positive" result should be obtained even in the verification for child CZ stored in the database by updating. In contrast, unless there is a special circumstance in which child CZ has been registered in the database as one of the children CA to CY for the reason that, for example, child CZ was a subject to a paternity test in a different case, the "negative" results should be obtained in the verification for the children CA to CY stored in the database.

That is, if a "positive" determination result has been obtained in the paternity test as a verification subject, child CZ stored in the database by updating serves as a positive control, in the verification step, whereas children CA to CY serve as a negative control unless the above-described special circumstance is present, so that it can be confirmed that the verification step itself is properly executed.

Conversely, if a "negative" determination result has been obtained in the paternity test as a verification subject, a "negative" result should be obtained even in the verification for child CZ stored in the database by updating.

That is, in the verification step, child CZ stored in the database by updating serves as a negative control, so that it can be confirmed that the verification step itself is properly executed.

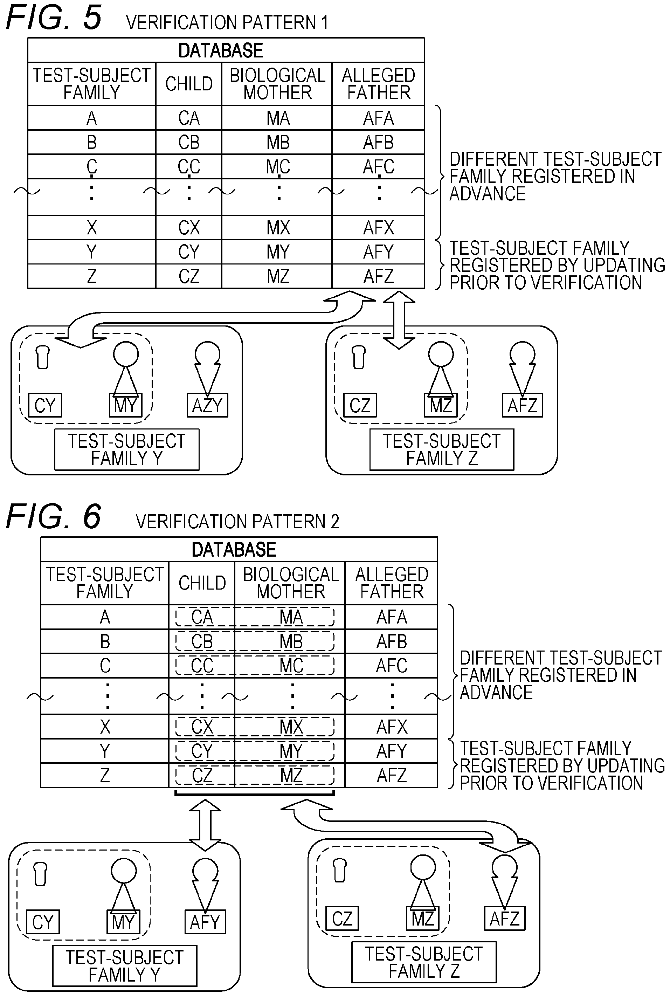

Next, an embodiment of executing verification pattern 1 is shown in Fig. 5. In this embodiment, the paternity tests (father-child test) performed on two sets of test-subject families, that is, specified test-subject family Y including three persons: child CY, the biological mother MY of child CY, and alleged father AFY, and specified test-subject family Z including three persons: a child CZ, the biological mother MZ of child CZ, and an alleged father AFZ, are the verification subjects in the verification step.

As a matter of course, the paternity test performed on one set of test-subject families may be used as the verification subject in the verification step. In addition, as shown in Fig. 5, the paternity test performed on two or more sets of test-subject families can be used as the verification subject in the verification step.

In the database, the genotyping results of children CA to CX, the biological mothers MA to MX of children CA to CX, and alleged fathers AFA to AFX belonging to different test-subject families A to X, which are different from specified test-subject families Y and Z, are stored, respectively, in advance (Fig. 5).

After completion of the genotyping of child CY, alleged father AFY, and mother MZ belonging to test-subject family Y, and the genotyping of child CZ, alleged father AFZ, and mother MZ belonging to test-subject family Z, the database is updated before execution of the verification step, and the genotyping results of child CY, alleged father AFY, and mother MY, and the genotyping results of child CZ, alleged father AFZ, and mother MZ are stored in the database (Fig. 5).

That is, the verification step is performed with reference to the database storing the genotyping results of children CA to CZ, alleged fathers AFA to AFZ, and mothers MA to MZ.

In the embodiment of Fig. 5, the paternity test is performed based on each of the genotyping results of child CY and mother MY and the genotyping results of alleged fathers AFA to AFZ stored in the database. That is, the paternity test (three-sample test) is performed for each of the combinations of child CY, mother MY, and alleged father AFA, child CY, mother MY, and alleged father AFB ... child CY, mother MY, and alleged father AFX, child CY, mother MY, and alleged father AFY, and child CY, mother MY, and alleged father AFZ.

In the embodiment of Fig. 5, independently of this, the paternity test is similarly performed based on each of the genotyping results of child CZ and mother MZ and the genotyping results of alleged fathers AFA to AFZ stored in the database.

Hereinafter, in order to avoid duplicate description, the description will be made focusing only on a case in which a paternity test is performed based on each of the genotyping results of child CZ, mother MZ, and alleged fathers AFA to AFZ. Needless to say, the following description also applies to a case in which a paternity test is performed based on each of the genotyping results of child CY, mother MY, and alleged fathers AFA to AFZ.

If the biological parent-child relationship between child CZ and any one of alleged fathers AFA to AFY was determined to be positive, it can be determined that the combination of the sample of child CZ, mother MZ, and alleged father AFZ treated as being taken from one specified test-subject family Z, was mixed up with a sample taken from a person belonging to a different test-subject family.

That is, it can be determined that the test results of the paternity test executed by analysis of the samples of child CZ, mother MZ, and alleged father AFZ belonging to test-subject family Z contain an error

Note that, in a case in which the above-described MC filter step is passed, it is not necessary to consider the mix-up of the sample of child CZ and the sample of mother MZ.

Furthermore, if a "positive" determination result has been obtained in the paternity test as a verification subject, a "positive" result should be obtained even in the verification for alleged father AFZ stored in the database by updating. In contrast, unless there is a special circumstance in which a person having a biological parent-child relationship with child CZ (including an ascendant and a descendant) has been registered in the database as any one of alleged fathers AFA to AFY, for the reason that, for example, the person was a subject of a paternity test in a different case, the "negative" result should be obtained in the verification on alleged fathers AFA to AFY stored in the database.

That is, if a "positive" determination result has been obtained in the paternity test as a verification subject, alleged father AFZ stored in the database by updating serves as a positive control, in the verification step, whereas alleged fathers AFA to AFY serve as a negative control unless the above-described special circumstance is present, so that it can be confirmed that the verification step itself is properly executed.

Conversely, if a "negative" determination result has been obtained in the paternity test as a verification subject, the "negative" result should be obtained even in the verification for alleged father AFZ stored in the database by updating.

That is, in the verification step, alleged father AFZ stored in the database by updating serves as a negative control, so that it can be confirmed that the verification step itself is properly executed.

Next, an embodiment of executing verification pattern 2 is shown in Fig. 6. The structures of test-subject families Y and Z and the data structures stored in the database in this embodiment are the same as those in the embodiment of Fig. 5.

In the embodiment of Fig. 6, the paternity test is performed based on the genotyping result of alleged father AFY and the genotyping result of each of children CA to CZ and mothers MA to MZ stored in the database. That is, a paternity test (three-sample test) is performed for each of the combinations of alleged father AFY, child CA, and mother MA, alleged father AFY, child CB, and mother MB, ... alleged father AFY, child CX, and mother MX, alleged father AFY, child CY, and mother MY, and alleged father AFY, child CZ, and mother MZ.

In the embodiment of Fig. 6, independently of this, the paternity test is similarly performed based on the genotyping result of alleged father AFZ and the genotyping results of children CA to CZ and mothers MA to MZ stored in the database.

Hereinafter, in order to avoid duplicate description, the description will be made focusing only on a case in which a paternity test is performed based on each of the genotyping results of alleged father AFZ, children CA to CZ, and mothers MA to MZ. Needless to say, the following description also applies to a case in which a paternity test is performed based on each of the genotyping results of alleged father AFY, children CA o CZ, and mothers MA to MZ.

If the biological parent-child relationship between alleged father AFZ and any one of children CA to CY was determined to be positive, it can be determined that the combination of samples of child CZ, mother MZ, and alleged father AFZ treated as being taken from a specified test-subject family Z was mixed up with a sample taken from a person belonging to a different test-subject family.

That is, it can be determined that the test results of the paternity test executed by analysis of the samples of child CZ, mother MZ, and alleged father AFZ belonging to test-subject family Z contain an error

Note that, in a case in which the above-described MC filter step is passed, it is not necessary to consider the mix-up of the sample of child CZ and the sample of mother MZ.

Furthermore, if a "positive" determination result has been obtained in the paternity test as a verification subject, a "positive" result should be obtained even in the verification for child CZ and mother MZ stored in the database by updating. In contrast, unless there is a special circumstance in which child CZ and mother MZ have been registered in the database as any of child CA to CY or mothers MA to MY, for the reason that, for example, child CZ and mother MZ were subjects for a paternity test in a different case, the "negative" results should be obtained in the verification for children CA to CY and mothers MA to MY stored in the database.

That is, if a "positive" determination result has been obtained in the paternity test as a verification subject, child CZ and mother MZ stored in the database by updating serves as a positive control, in the verification step, whereas children CA to CY and the mothers MA to MY serve as a negative control unless the above-described special circumstance is present, so that it can be confirmed that the verification step itself is properly executed.

Conversely, if a "negative" determination result has been obtained in the paternity test as a verification subject, the "negative" result should also be obtained in the verification for child CZ and mother MZ stored in the database by updating.

That is, in the verification step, child CZ and mother MZ stored in the database by updating serve as a negative control, so that it can be confirmed that the verification step itself is properly executed.

Next, the effectiveness of verification common to the embodiments shown in Figs. 3 to 6 will be described.

In the embodiments shown in Figs. 3 to 6, the paternity tests for test-subject families Y and Z are verification subjects. Here, it is assumed that a sample mix-up occurs between test-subject families Y and Z.

At this time, the following four cases are conceivable as the result of the paternity test of each of test-subject families X and Y.

Case 1: A positive result of the biological parent-child relationship was obtained between a child and an alleged father in both paternity tests for test-subject families Y and Z.

Case 2: A positive result of the biological parent-child relationship was obtained in the paternity test for test-subject family Y, while a negative result of the biological parent-child relationship was obtained in the paternity test for test-subject family Z.

Case 3: A negative result of the biological parent-child relationship was obtained in the paternity test for test-subject family Y, while a positive result of the biological parent-child relationship was obtained in the paternity test for test-subject family Z.

Case 4: A negative result of the biological parent-child relationship was obtained in both paternity tests for test-subject families Y and Z

In the cases 1 to 3 described above, it is possible to find that a sample mix-up occurred by passing through the verification step.

However, in the case corresponding to Case 4, in which actually no biological parent-child relationship exists between child CY and alleged father AFY and between child CZ and alleged father AFZ, it cannot be found that a sample mix-up occurred even after the verification step. However, in this case, the DNA profile of the test subject caused by a sample mix-up contains a mistake, but the final determination result that denies a biological parent-child relationship is still the same.

The embodiments shown in Figs. 3 to 6 relate to a father-child test. However, the invention is not limited to the father-child test, and, needless to say, can be applied to the mother-child test.

In the case of application to the mother-child test, the "alleged father" in the embodiments shown in Figs. 3 to 6 can be replaced with the "alleged mother" and then applied. In addition, in the embodiments shown in Figs. 5 and 6, "biological mother" can be replaced with "biological father" and then applied.

The verification method of the invention can be applied to both the paternity test performed before birth and the paternity test performed after birth.

If the paternity test to which the verification method of the invention is applied, is performed before birth, the test may have the MC filter step, as described above.

Also if the paternity test to which the verification method of the invention is applied, is performed after birth, the same filter as the above-described MC filter can be employed. Hereinafter, two cases, a father-child test and a mother-child test, will be described.

In a paternity test (father-child test) performed by analysis of samples collected from three persons: a child after birth, the biological mother of the child, and an alleged father, the test preferably contains a post-natal MC filter step defined below.

<Postnatal MC Filter>

The genotyping of a child based on a sample containing a nucleic acid having genetic information of the child collected from the child after birth,

the genotyping of the biological mother of the child based on a sample containing a nucleic acid having genetic information of the mother and collected from the mother are performed and
if the following (i) is observed in a predetermined number or more, the paternity test to be performed based on the collected samples is stopped without executing.

(i) A polymorphic locus that is a homozygote of alleles different in genotype between the mother and the child.

If the postnatal MC filter is provided, it becomes unnecessary for a witness to attend when a sample is collected from a biological mother, even in a legal test.

In addition, in a paternity test (mother-child test) performed by analyzing samples collected from a child after birth, the biological father of the child, and an alleged mother, a post-natal FC filter step defined below is preferably included.

<Postnatal FC filter>

The genotyping of a child after birth based on a sample containing a nucleic acid having genetic information of the child, taken from the child, and

the genotyping of a biological father based on a sample containing a nucleic acid having genetic information of the father and collected from the father
are performed and if the following (i) is observed in a predetermined number or more, the paternity test to be performed based on the collected samples is stopped without executing.

(i) A polymorphic locus that is a homozygote of alleles different in genotype between the father and the child.

If the postnatal MC filter is provided, it becomes unnecessary for a witness to attend when a sample is collected from a biological mother, even in a legal test.
As described above, the verification method of the invention is not limited to a paternity test, and can be applied to the verification of all blood relationships.
The "alleged father" and the "child" in Figs. 3 to 4 can be rephrased with an arbitrary "alleged blood relative" and "specified person", respectively, depending on the structure of the test family for the blood-relationship test as a verification subject, and then applied.
The "alleged father", the "child", and the "biological mother" in Figs. 5 to 6 can be rephrased with an arbitrary "alleged blood relative", "specified person", and "biological blood relative", respectively, depending on the structure of the test family for the blood-relationship test as a verification subject, and then applied.

<3> Specific Embodiment of Paternity Test

The paternity testing method employing the detection method of the invention will be described again with reference to Figs. 1 and 2.
The embodiment shown in Fig. 1 includes a two-sample testing step based on samples of two persons obtained from a specified test-subject family including an alleged father and a child. In such an embodiment, first, the samples of two persons: an alleged father and a child, are analyzed to obtain the genotyping results of the alleged father and the child.
In the database to be referred to in the verification step, the genotyping results of a different test-subject family are stored in advance. This embodiment includes storing the genotyping results of two persons belonging to a specified test-subject family in the database and updating the database after completion of genotyping (Fig. 1).
Note that, in a case in which two or more sets of test-subject families for the verification step are present, these may be registered at a time, thereby updating the database, simultaneously. In this case, the updated database stores the genotyping results of children CA to CZ and alleged fathers AFA to AFZ, as shown in Figs. 3 to 6.
After completion of the genotyping of two persons belonging to a specified test-subject family, a two-sample testing step is executed based on the results (Fig. 1). In this embodiment, the verification step is executed regardless of the results of the two-sample testing step.
When the verification step is executed, the verification is performed with reference to the updated database and according to verification pattern 1 and/or verification pattern 2.
If description is made in more detail according to the embodiments in which there are two sets of test-subject families as a verification subject, as shown in Figs. 3 to 6, verification is performed according to the following verification pattern 1-1 and/or verification pattern 2-1, and verification pattern 1-2 and/or verification pattern 2-2, in the verification step.

(Verification Pattern 1-1)

Based on the genotyping result of child CY, and

the genotyping result of each of alleged fathers AFA to AFZ stored in the updated database,
a paternity test is performed on a combination of child CY and each of alleged fathers AFA to AFZ.

(Verification Pattern 2-1)

Based on the genotyping result of alleged father AFY, and

the genotyping result of each of children CA to CZ stored in the updated database,
a paternity test is performed on a combination of alleged father AFY and each of children CA to CZ.

(Verification Pattern 1-2)

Based on the genotyping result of child CZ, and

the genotyping result of each of alleged fathers AFA to AFZ stored in the updated database,
a paternity test is performed on a combination of child CZ and each of alleged fathers AFA to AFZ.

(Verification Pattern 2-2)

Based on the genotyping result of alleged father AFZ, and

the genotyping result of each of children CA to CZ stored in the updated database,
a paternity test is performed on a combination of alleged father AFZ and each of children CA to CZ.

That is, in this embodiment, the genotyping results of the specified test-subject family reflected in the updated database are regarded as the genotyping results of a different test-subject family, and verification is performed according to verification pattern 1 and/or verification pattern 2.

Hereinafter, in order to avoid duplicate description, the description will be made according to an embodiment in which verification patterns 1-2 and/or 2-2 are executed. Note that the following description naturally applies to an embodiment in which verification patterns 1-1 and/or 2-1 are executed.

If a biological parent-child relationship is confirmed to be positive by the two-sample testing step, a "positive" result should be provided in the paternity test (verification pattern 1-2) based on the genotyping results of child CZ and alleged father AFZ stored in the updated database, and the paternity test (verification pattern 2-2) based on the genotyping results of alleged father AFZ and child CZ stored in the updated database, in the verification step.

If a biological parent-child relationship is confirmed to be negative by the two-sample testing step, a "negative" result should be provided in the paternity test (verification pattern 1-2) based on the genotyping results of child CZ and alleged father AFZ stored in the updated database, and the paternity test (verification pattern 2-2) based on the genotyping results of alleged father AFZ and child CZ stored in the updated database, in the verification step.

That is, the updated database always has a positive control bringing a "positive" result or a negative control bringing a "negative" result. As a result, whether or not an error occurs in the verification step itself can be confirmed.

The embodiment shown in Fig. 2 includes a three-sample testing step based on samples of three persons taken from a specified test-subject family including an alleged father, a child, and the biological mother of the child. This embodiment includes an MC filter step (Fig. 2). After completion of the genotyping of three persons belonging to a specified test-subject family, the result is stored in a database and updated (Fig. 2).

Note that, in a case in which two or more sets of test-subject families for the verification step are present, these may be registered at a time, thereby updating the database, simultaneously. In this case, the updated database stores the genotyping results of children CA to CZ and alleged fathers AFA to AFZ, as shown in Figs. 3 to 6.

In this embodiment, the verification is performed with reference to the updated database and according to verification pattern 1 and/or verification pattern 2, in the verification step.

If description is made in more detail according to the embodiments in which there are two sets of test-subject families as a verification subject, as shown in Figs. 3 to 6, verification is performed according to the following verification pattern 1-1 and/or verification pattern 2-1, and verification pattern 1-2 and/or verification pattern 2-2, in the verification step.

(Verification Pattern 1-1)

Based on the genotyping result of child CY,

optionally, the genotyping result of mother MY, and
the genotyping result of each of alleged fathers AFA to AFZ stored in the updated database,
a paternity test is performed on a combination of child CY and each of alleged fathers AFA to AFZ.

(Verification Pattern 2-1)

Based on the genotyping result of alleged father AFY,

optionally, the genotyping result of each of mothers MA to MZ stored in the updated database, and
the genotyping result of each of children CA to CZ stored in the updated database,
a paternity test is performed on a combination of alleged father AFY and each of children CA to CZ.

(Verification Pattern 1-2)

Based on the genotyping results of child CZ,

optionally, the genotyping result of mother MZ, and
the genotyping result of each of alleged fathers AFA to AFZ stored in the updated database,
a paternity test is performed on a combination of child CZ and each of alleged fathers AFA to AFZ.

(Verification Pattern 2-2)

Based on the genotyping result of alleged father AFZ,

optionally, the genotyping result of each of mothers MA to MZ stored in the updated database, and
the genotyping result of each of children CA to CZ stored in the updated database,
a paternity test is performed on a combination of alleged father AFZ and each of children CA to CZ.

Similarly to the embodiment of Fig. 1, in the embodiment of Fig. 2, the genotyping results of the specified test-subject family reflected in the updated database are regarded as the genotyping results of a different test-subject family, and verification is performed according to verification pattern 1 and/or verification pattern 2.

In the embodiment of Fig. 2, similarly to the embodiment of Fig. 1, the updated database has a positive control bringing a "positive" result if the corresponding test-subject family having a positive result is present, or has a negative control bringing a "negative" result. As a result, whether or not an error occurs in the verification step itself can be confirmed.

Industrial Applicability

The invention can be applied to a paternity test.

**Claims**

1. A non-invasive prenatal paternity testing method, which is
a non-invasive prenatal paternity testing method using maternal blood, comprising a two-sample testing step and/or a three-sample testing step defined below:

    \<Two-sample Testing Step\>

    A step of determining whether or not a biological parent-child relationship exists between an alleged father and a fetus based on genotyping results of two persons: the alleged father and the fetus, at a plurality of polymorphic loci,
with the proviso that the genotyping of the fetus is performed according to a determination method based on criterion a, defined below:

    (Determination Method Based on Criterion a)
Provided that
the allele which is contained in data obtained by analysis of a circulating cell-free nucleic acid sample collected from a pregnant mother, derived from a specified polymorphic locus and detected with the strongest signal intensity, is denoted as allele A, and
the allele detected with the same signal intensity as that of allele A or the second strongest signal intensity is denoted as allele B,
determination is performed using the ratio of the signal intensity (Fa) of allele A and the ratio of the signal intensity (Fb) of allele B with respect to the combined intensity of signals derived from the specified polymorphic locus, as indexes, and according to the following criterion a:
\<Criterion a\>

· If 0% ≤ Fb {≤, <} L% is satisfied, the genotype at the specified polymorphic locus of a fetus is determined to be AA homozygote

· If M% {≤, <} Fb {≤, <} N% is satisfied, the genotype at the specified polymorphic locus of a fetus is determined to be AB heterozygote

· If Fa = Fb is satisfied, the genotyping of the specified polymorphic locus is skipped without being performed

· If Fb does not satisfy any of the above conditions, the genotyping of the specified polymorphic locus is skipped without being performed

(N is a numerical value of less than 50 and satisfies the relational expression 0 {≤, <} L < M {≤, <} N. Note that the notation of {≤, <} indicates that either one of the inequality signs, ≤ and <, can be arbitrarily selected);

<Three-sample Testing Step>

A step of determining whether or not a biological parent-child relationship exists between an alleged father and a fetus based on genotyping results of three persons: a biological mother, the alleged father, and the fetus, at a plurality of polymorphic loci,
with the proviso that the genotyping of the fetus is performed according to a determination method based on criterion a or a determination method based on criterion b, defined below:

(Determination Method Based on Criterion b)
Provided that
the allele which is contained in data obtained by analysis of a circulating cell-free nucleic acid sample, derived from a specified polymorphic locus determined as a homozygote in the mother, and detected with the strongest signal intensity, is denoted as allele A, and
the allele which is detected with the same signal intensity as allele A or the second strongest signal intensity is denoted as allele B,
determination is performed using the ratio of the signal intensity (Fa) of allele A and the ratio of the signal intensity (Fb) of allele B with respect to the combined intensity of signals derived from the specified polymorphic locus, as indexes, and according to the following criterion b:
<Criterion b>

· If 0% ≤ Fb {≤, <} L% is satisfied, the genotype at the specified polymorphic locus of a fetus is determined to be AA homozygote

· if M% {≤, <} Fb is satisfied, then the genotype at the specified polymorphic locus of the fetus is determined to be AB heterozygote

· If Fa = Fb is satisfied, the genotyping of the specified polymorphic locus is skipped without being performed

· If Fb does not satisfy any of the above conditions, the genotyping of the specified polymorphic locus is skipped without being performed

(the relational expression of 0 {≤, <} L < M is satisfied. Note that the notation of {≤, <} indicates that either one of the inequality signs, ≤ and <, can be arbitrarily selected).

2. The non-invasive prenatal paternity testing method according to claim 1, wherein testing whether or not a biological parent-child relationship exists, comprises

obtaining a combined paternity index (CPI) by multiplying paternity indexes (PI) obtained for individual polymorphic loci, thereby determining "positive", "negative", or "undeterminable" based on a criterion determined in advance according to a numerical value of the CPI,
PI is obtained based on the following Table 1 in the two-sample testing step, and
PI is obtained based on the following Table 1 or Table 2, in the three-sample testing step.

[Table 1]

| Case | Genotype | | Paternity Index |
| --- | --- | --- | --- |
| | Alleged Father | Fetus | PI |
| (I) | AA | AA | 1/a |
| (II) | AA | AB | kb |
| (III) | BB | AA | k0 |
| (IV) | BB | AB | 1/b |
| (V) | AB | AA | 0.5/a |
| (VI) | AB | AB | 0.5/b |

[Table 2]

| Case | Genotype | | | Paternity Index |
| --- | --- | --- | --- | --- |
| | Mother | Alleged Father | Fetus | PI |
| (I) | AA | AA | AA | 1/a |
| (II) | AA | AA | AB | kb |
| (III) | AA | BB | AA | k0 |
| (IV) | AA | BB | AB | 1/b |
| (V) | AA | AB | AA | 0.5/a |
| (VI) | AA | AB | AB | 0.5/b |

(In Tables 1 and 2, an allele having a relatively high signal intensity is denoted as A, and an allele having a relatively low signal intensity therein is denoted as B, in the analysis of a circulating cell-free nucleic acid sample. In Table 1, k0 is a false negative rate, kb is a false positive rate, a is an appearance frequency of A, and b is an appearance frequency of B.)

3. The non-invasive prenatal paternity testing method according to claim 1 or 2, comprising the three-sample testing step and an MC filter step defined below.
   <MC Filter Step>

   Genotyping of the mother is performed based on a sample containing a nucleic acid having genetic information of the mother, collected from the mother, and substantially not containing a nucleic acid having genetic information of the fetus, and
   if

   (i) the polymorphic locus which is a homozygote of alleles mutually different in genotype between the mother and the fetus or circulating cell-free nucleic acid,
   and/or
   (ii) the polymorphic locus in which the genotype in the mother is a heterozygote and the genotype of the circulating cell-free nucleic acid is a homozygote,

   are observed in a predetermined number or more, the paternity test to be performed based on the collected samples is stopped without executing.

4. The non-invasive prenatal paternity testing method according to claim 3, wherein genotyping for identifying a genotype of the circulating cell-free nucleic acid is performed by a method defined below.
   <Genotyping of Circulating Cell-free Nucleic Acid>

   Provided that

   an allele which is contained in data obtained by analysis of the circulating cell-free nucleic acid sample, derived from a specified polymorphic locus, and detected with the strongest signal intensity is denoted as allele A, and

an allele detected with the same signal intensity as allele A or the second strongest signal intensity is denoted as allele B,

genotyping is performed using the ratio of the signal intensity (Fa) of allele A and the ratio of the signal intensity (Fb) of allele B with respect to the combined intensity of signals derived from the specified polymorphic locus, as indexes, and according to the following criterion "c".

<Reference c>

· If $X \{\leq, <\} Fa \{\leq, <\} Y\%$ is satisfied, the genotype at the specified polymorphic locus of the circulating cell-free nucleic acid is determined to be an AB heterozygote.
· If $Z\% \{\leq, <\} Fa \leq 100$ is satisfied, the genotype at the specified polymorphic locus of the circulating cell-free nucleic acid is determined to be an AA homozygote.
· If $Fa = Fb$ is satisfied, the genotype of the specified polymorphic locus is an AB heterozygote.
(the relational expression of $X \{\leq, <\} Y < Z$ is satisfied. Note that the notation of $\{\leq, <\}$ indicates that either one of the inequality signs, $\leq$ and $<$, can be arbitrarily selected).

5. The non-invasive prenatal paternity testing method according to claim 1 or 2, wherein a verification step defined below is executed based on a sample collected from a specified test-subject family.

<Verification Step>
In order to verify an erroneous determination caused by a mix-up of a sample with a sample, which is collected from a test-subject family different from the specified test-subject family, verification is performed according to verification pattern 1 and/or verification pattern 2 defined below.
(Verification Pattern 1)

Based on a genotyping result of the fetus belonging to the specified test-subject family, and
optionally, a genotyping result of the mother belonging to the specified test-subject family,
a genotyping result of an alleged father belonging to the different test-subject family,
a paternity test is performed on a combination of the alleged father and the fetus.

(Verification Pattern 2)

Based on a genotyping result of a fetus belonging to the different test-subject family,
optionally, a genotyping result of the biological mother of the fetus belonging to the different test-subject family and
a genotyping result of the alleged father belonging to the specified test-subject family,
a paternity test is performed on a combination of the alleged father and the fetus.

6. The non-invasive prenatal paternity testing method according to claim 5, wherein, in the verification step,
the verification is performed according to verification pattern 1 and/or verification pattern 2 with reference to a database storing genotyping results of an alleged father, a fetus, and optionally a biological mother of the fetus belonging to one or two or more of the different test-subject families.

7. The non-invasive prenatal paternity testing method according to claim 6, comprising, after completion of genotyping of the alleged father, the fetus, and optionally the mother belonging to the specified test-subject family, storing results in the database and updating the database, and

executing the verification step with reference to the updated database, in the verification step,
wherein,
in the verification step, the verification according to verification pattern 1 and/or verification pattern 2 is performed by regarding the genotyping results of the specified test-subject family reflected in the database, as the genotyping results of the different test-subject family.

8. The non-invasive prenatal paternity test according to claim 5, wherein the genotyping of the alleged father and the genotyping of the fetus are

executed at mutually different places and/or different times, and/or executed by mutually different operators.

# FIG. 1

```
┌─────────────────────┐                        ┌─────────────────────┐
│ PERFORM GENOTYPING  │                        │ PERFORM GENOTYPING  │
│  OF ALLEGED FATHER  │                        │      OF CHILD       │
└─────────────────────┘                        └─────────────────────┘
              │                                              │
              └──────────────┬───────────────────────────────┘
                   ┌──────────────────────┐
                   │ TWO-SAMPLE TEST STEP │
                   └──────────────────────┘
                              │
┌──────────┐   N      ╱ IS THERE        ╲   Y   ┌──────────┐
│ POSITIVE │──────────  PARENT-CHILD RELATIONSHIP ──────│ POSITIVE │
└──────────┘           ╲   OR NOT?      ╱        └──────────┘
                              │
┌─────────────────┐   ┌────────────────────┐
│ UPDATE DATABASE │───│ VERIFICATION STEP  │
└─────────────────┘   └────────────────────┘
                              │
                      ╱ IS THERE MIX-UP WITH ╲   N   ┌──────────────────┐
                        SAMPLES?               ──────│ RESULT OF TEST STEP │
                      ╲                      ╱        │    IS RELIABLE   │
                              │ Y                     └──────────────────┘
                   ┌────────────────────┐
                   │ RESULT OF TEST STEP│
                   │   IS NOT RELIABLE  │
                   └────────────────────┘
```

# FIG. 2

```
                   ┌─────────────────────┐
                   │ PERFORM GENOTYPING  │
                   │     OF MOTHER       │
                   └─────────────────────┘
┌─────────────────────┐                    ┌─────────────────────┐
│ PERFORM GENOTYPING  │                    │ PERFORM GENOTYPING  │
│  OF ALLEGED FATHER  │                    │     OF FETUS        │
└─────────────────────┘                    └─────────────────────┘
                   ┌──────────────────┐
                   │  MC FILTER STEP  │
                   └──────────────────┘
                              │
                      ╱ IS THERE MIX-UP WITH ╲   Y   ┌──────┐
                        SAMPLES?               ──────│ END  │
                      ╲                      ╱        └──────┘
                              │ N
                   ┌────────────────────────┐
                   │ THREE-SAMPLE TEST STEP │
                   └────────────────────────┘
                              │
┌──────────┐   N      ╱ IS THERE        ╲   Y   ┌──────────┐
│ NEGATIVE │──────────  PARENT-CHILD RELATIONSHIP ──────│ POSITIVE │
└──────────┘           ╲   OR NOT?      ╱        └──────────┘
                              │
┌─────────────────┐   ┌────────────────────┐
│ UPDATE DATABASE │───│ VERIFICATION STEP  │
└─────────────────┘   └────────────────────┘
                              │
                      ╱ IS THERE MIX-UP WITH ╲   N   ┌──────────────────┐
                        SAMPLES?               ──────│ RESULT OF TEST STEP │
                      ╲                      ╱        │    IS RELIABLE   │
                              │ Y                     └──────────────────┘
                   ┌────────────────────┐
                   │ RESULT OF TEST STEP│
                   │   IS NOT RELIABLE  │
                   └────────────────────┘
```

# FIG. 3

VERIFICATION PATTERN 1

| DATABASE | | |
|---|---|---|
| TEST-SUBJECT FAMILY | CHILD | ALLEGED FATHER |
| A | CA | AFA |
| B | CB | AFB |
| C | CC | AFC |
| : | : | : |
| X | CX | AFX |
| Y | CY | AFY |
| Z | CZ | AFZ |

DIFFERENT TEST-SUBJECT FAMILY REGISTERED IN ADVANCE

TEST-SUBJECT FAMILY REGISTERED BY UPDATING PRIOR TO VERIFICATION

CY  AFY

TEST-SUBJECT FAMILY Y

CY  AFY

TEST-SUBJECT FAMILY Z

## FIG. 4

VERIFICATION PATTERN 2

| DATABASE | | |
|---|---|---|
| TEST-SUBJECT FAMILY | CHILD | ALLEGED FATHER |
| A | CA | AFA |
| B | CB | AFB |
| C | CC | AFC |
| ⋮ | ⋮ | ⋮ |
| X | CX | AFX |
| Y | CY | AFY |
| Z | CZ | AFZ |

DIFFERENT TEST-SUBJECT FAMILY REGISTERED IN ADVANCE

TEST-SUBJECT FAMILY REGISTERED BY UPDATING PRIOR TO VERIFICATION

| CY | AFY |
|---|---|

TEST-SUBJECT FAMILY Y

| CY | AFY |
|---|---|

TEST-SUBJECT FAMILY Z

## FIG. 5  VERIFICATION PATTERN 1

| DATABASE | | | |
|---|---|---|---|
| TEST-SUBJECT FAMILY | CHILD | BIOLOGICAL MOTHER | ALLEGED FATHER |
| A | CA | MA | AFA |
| B | CB | MB | AFB |
| C | CC | MC | AFC |
| ⋮ | ⋮ | ⋮ | ⋮ |
| X | CX | MX | AFX |
| Y | CY | MY | AFY |
| Z | CZ | MZ | AFZ |

DIFFERENT TEST-SUBJECT FAMILY REGISTERED IN ADVANCE

TEST-SUBJECT FAMILY REGISTERED BY UPDATING PRIOR TO VERIFICATION

CY   MY   AZY

TEST-SUBJECT FAMILY Y

CZ   MZ   AFZ

TEST-SUBJECT FAMILY Z

## FIG. 6  VERIFICATION PATTERN 2

| DATABASE | | | |
|---|---|---|---|
| TEST-SUBJECT FAMILY | CHILD | BIOLOGICAL MOTHER | ALLEGED FATHER |
| A | CA | MA | AFA |
| B | CB | MB | AFB |
| C | CC | MC | AFC |
| ⋮ | ⋮ | ⋮ | ⋮ |
| X | CX | MX | AFX |
| Y | CY | MY | AFY |
| Z | CZ | MZ | AFZ |

DIFFERENT TEST-SUBJECT FAMILY REGISTERED IN ADVANCE

TEST-SUBJECT FAMILY REGISTERED BY UPDATING PRIOR TO VERIFICATION

CY   MY   AFY

TEST-SUBJECT FAMILY Y

CZ   MZ   AFZ

TEST-SUBJECT FAMILY Z

## FIG. 7

## INTERNATIONAL SEARCH REPORT

| International application No. |
| --- |
| **PCT/JP2024/032774** |

### A. CLASSIFICATION OF SUBJECT MATTER

*C12Q 1/6809*(2018.01)i; *G16H 10/40*(2018.01)i
FI: C12Q1/6809 Z; G16H10/40

According to International Patent Classification (IPC) or to both national classification and IPC

### B. FIELDS SEARCHED

Minimum documentation searched (classification system followed by classification symbols)

C12Q1/00-3/00; G16H10/40

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| A | WO 2022/131328 A1 (SEEDNA INC.) 23 June 2022 (2022-06-23)<br>claim 1, paragraphs [0001], [0003], [0008] | 1-8 |
| A | JP 2014-502845 A (NATERA, INC.) 06 February 2014 (2014-02-06)<br>claims 1, 4, 16 | 1-8 |
| A | WO 2017/082034 A1 (FUJIFILM CORPORATION) 18 May 2017 (2017-05-18)<br>claim 1, paragraphs [0003], [0004] | 1-8 |
| A | JP 2023-516299 A (LABORATORY CORPORATION OF AMERICA HOLDINGS) 19 April 2023 (2023-04-19)<br>claim 1 | 1-8 |
| P, A | JP 7331325 B1 (SEEDNA INC.) 23 August 2023 (2023-08-23)<br>entire text | 1-8 |

☐ Further documents are listed in the continuation of Box C.    ☑ See patent family annex.

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **20 November 2024** | **03 December 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/032774**

| Patent document cited in search report | | | Publication date (day/month/year) | Patent family member(s) | | | Publication date (day/month/year) |
|---|---|---|---|---|---|---|---|
| WO | 2022/131328 | A1 | 23 June 2022 | US | 2023/0227897 | A1 | |
| | | | | claim 1, paragraphs [0001], [0004], [0014] | | | |
| | | | | EP | 4266315 | A1 | |
| | | | | KR | 10-2023-0012033 | A | |
| JP | 2014-502845 | A | 06 February 2014 | US | 2012/0122701 | A1 | |
| | | | | claims 1, 4, 16 | | | |
| | | | | WO | 2012/088456 | A2 | |
| | | | | EP | 2656263 | A2 | |
| | | | | CN | 103608466 | A | |
| WO | 2017/082034 | A1 | 18 May 2017 | US | 2018/0247019 | A1 | |
| | | | | claim 1, paragraphs [0004], [0005] | | | |
| | | | | EP | 3375886 | A1 | |
| JP | 2023-516299 | A | 19 April 2023 | US | 2023/0120825 | A1 | |
| | | | | claim 1 | | | |
| | | | | WO | 2021/174079 | A2 | |
| | | | | EP | 4110953 | A2 | |
| JP | 7331325 | B1 | 23 August 2023 | JP | 2024-35014 | A | |
| | | | | WO | 2024/047977 | A1 | |
| | | | | CN | 117980504 | A | |

Form PCT/ISA/210 (patent family annex) (July 2022)

33

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014502845 A